# EUROPÄISCHE PATENTSCHRIFT

(11) **EP 3 326 670 B1**
(45) Veröffentlichungstag und Bekanntmachung des Hinweises auf die Patenterteilung: **03.05.2023**
(21) Anmeldenummer: 17203284.9
(22) Anmeldetag: 23.11.2017
(51) Int. Cl.: A61M 1/36

(54) **MEDIZINTECHNISCHER LUFTABSCHEIDER ZUR ANWENDUNG BEI BLUTBEHANDLUNGEN**
MEDICAL AIR SEPARATOR FOR USE IN BLOOD TREATMENT
SÉPARATEUR MÉDICAL D'AIR DESTINÉ À L'UTILISATION DANS DES TRAITEMENTS DU SANG

(30) Priorität: 24.11.2016 DE 102016122660
(43) Veröffentlichungstag der Anmeldung: 30.05.2018
(73) Patentinhaber: B. Braun Avitum AG, 34212 Melsungen (DE)
(72) Erfinder: Ritter, Kai-Uwe, 91126 Rednitzhembach (DE)
(74) Vertreter: Winter, Brandl - Partnerschaft mbB

(56) Entgegenhaltungen:
- DE-A1-102009 030 283
- US-A1- 2006 029 514
- US-A1- 2009 199 708
- US-A1- 2013 053 693
- US-A1- 2015 157 782

## Beschreibung

Die Erfindung betrifft einen medizintechnischen Luftabscheider zum Abscheiden von Gasblasen sowie Luftblasen, insbesondere von Mikroblasen, aus durch den Luftabscheider hindurchgeleitetem Blut. Der Luftabscheider weist eine im Wesentlichen hohlzylinderförmige Luftabscheidekammer mit einem in Längsrichtung einerseits der Luftabscheidekammer angeordneten Bluteinlass und einem in Längsrichtung andererseits ausgebildeten Blutauslass auf, wobei die Luftabscheidekammer derart beschaffen ist, dass eine im Wesentlichen spiralförmig um eine Längsachse der Kammer rotierende Blutströmung vom Bluteinlass zum Blutauslass ausbildbar ist. Die Erfindung betrifft außerdem ein Blutschlauchsystem und eine Vorrichtung zur extrakorporalen Blutbehandlung mit einem erfindungsgemäßen Luftabscheider.

### Hintergrund der Erfindung

Mikroblasen im Blut sind ein nur schwer zu vermeidender und stetiger Begleiteffekt bei extrakorporalen Blutbehandlungen, wie zum Beispiel der Dialyse. Derzeit wird unter Fachleuten diskutiert, ob und in wie weit sie gesundheitsschädlich sind. In einer überarbeiten Ausgabe der Norm 60601-2-16 wurde deshalb erstmals ein Grenzwert für Mikroblasen aufgenommen.

Nach einer Veröffentlichung mit dem Titel "Microbubbles of air may occur in the organs of hemodialysis patients" der Autoren B. Stegmavr, T. Brännström, U. Forsberq, P. Jonson, C. Steqmayr und J. Hultdin (siehe www.ncbi.nlm.nih.gov/pubmed/22236622) wurde im Rahmen einer Studie verifiziert, dass Mikroblasen während einer Hämodialyse in die Blutbahn und die Lunge eines Patienten gelangen können. Außerdem passieren Mikroblasen die pulmonalen Kapillaren, dringen in den arteriellen Bereich des Körpers ein und verteilen sich durch den Körper. Resultat können Organschäden und schlechte Prognosen bei Hämodialysepatienten sein. Die Daten stützen die Wichtigkeit, Mikroblasen in extrakorporalen Blutkreisläufen zu verringern.

Eine weitere Veröffentlichung der Autoren S. Wagner, C. Rode, R. Wojke und B. Canaud (siehe: http://www.ncbi.nlm.nih.g0v/pmc/articles/PMC4515906/) mit dem Titel "Observation of microbubbles during standard dialysis treatments" befasst sich mit Ursachen für die Bildung derartiger Mikroblasen. Bei den Untersuchungen kam heraus, dass einige mögliche Quellen für Mikroblasen identifiziert werden konnten, wie arterielle Luer-Lock-Konnektoren bei negativem Druck und restliche Blasen infolge eines unzureichenden Primings. Allerdings konnten nicht alle Ursachen für Mikroblasen ermittelt werden, die gleichwohl bei nahezu allen Behandlungsformen, wie bspw. Hämodialyse und Online-Hämodiafiltration auftreten. Im Allgemeinen hängt die Mikroblasenrate sowohl mit der Strömungsrate (Korrelationskoeffizient von 0,45) und einem negativen arteriellen Druck (Korrelationskoeffizient 0,67) zusammen.

Nach einer Veröffentlichung von P. Laird (siehe: http://www.hemodoc.info/20l2/0l/cognitive-impairment-on-dialysis-the-microbubble-connection.htmlCognitive Impairment on Dialysis: The Microbubble Connection) stellen kognitive Beeinträchtigungen eine bekannte Komplikation bei Hämodialysepatienten dar, wobei wenig über deren Ursachen bekannt ist. Eine mögliche Ursache könnten Mikroembolien sein, die während einer Hämodialyse auftreten, die Lungenbarriere passieren und ischämische Läsionen von Organen wie dem Gehirn hervorrufen können.

### Stand der Technik

Zum Entfernen von Luft aus einem Blutstrom während einer extrakorporalen Blutbehandlung sind Luftabscheider bekannt, die in eine Vorrichtung zur extrakorporalen Blutbehandlung integriert sind. Diese Luftabscheider weisen eine Luftabscheidekammer auf, in die Blut mit einer seitlichen oder tangentialen Einströmrichtung eingeleitet wird. Infolge der seitlichen Einströmung wird in der Kammer ein Wirbel/Strudel erzeugt. Das Blut strömt in einer spiralförmigen oder wendelförmigen Bahn vom Bluteinlass der Kammer zu einem Blutauslass. Infolge der bei einem derartigen Strömungsbild wirkenden Zentrifugalkräfte bzw. Zentripetalkräfte wird Blut nach radial außen gedrückt, während Luft und damit auch Luft- oder Gasblasen in der Mitte der rotierenden Strömung und der Kammer bleiben. Derart kann Luft bzw. können Luftblasen aus dem Blut austreten und entfernt werden.

Der Luftabscheider nach DE 10 2009 030 283 A1 verzichtet gänzlich auf eine Rotation der Flüssigkeit in der Luftabscheidekammer. Die Luftabscheidekammer ist vielmehr so konstruiert, dass Gasblasen aus der Flüssigkeit von alleine aufsteigen.

Bei diesen bekannten Luftabscheidern (und Verfahren zur Luftabscheidung) ist von Nachteil, dass sich Luftblasen vor allem bei niedrigen Flussraten gut abscheiden, bei höheren Flussraten hingegen nicht oder nur in unzureichenden Maß. Als Grund wird vermutet, dass die Verweildauer in der Kammer nicht ausreicht, um den Luftblasen genügend Zeit zum Aufsteigen zu geben. Eine dem Grunde nach ausreichende Wirkung des Strudels scheint vorhanden zu sein, um die Abscheidung der Luftblasen zu unterstützen. Bei höheren Flussraten ist diese Wirkung stärker, infolge der kürzeren Verweilzeit aber dennoch begrenzt und unzureichend.

Vor diesem Hintergrund zeigen US 2006/0029514 A1, US 2009/0199708 A1, US 2015/0157782 A1 und US 2013/0053693 A1 jeweils einen Luftabscheider mit einer Luftabscheidekammer, innerhalb derer ein Rührelement angeordnet ist, um eine Rotation der Flüssigkeit innerhalb der Luftabscheidekammer zu erzeugen.

### Kurzbeschreibung der Erfindung

Ausgehend von dem vorstehend beschriebenen Stand der Technik liegt der vorliegenden Erfindung die Aufgabe zugrunde, die zuvor angeführten Nachteile zu beseitigen, insbesondere einen Luftabscheider für eine Verwendung im Rahmen einer extrakorporalen Blutbehandlung zu schaffen, der eine einfache und zuverlässige Entfernung von Gas-und Luftblasen aus Blut ermöglicht und insbesondere den Prozess der Luftabscheidung von Mikroblasen verbessert.

Nach der Erfindung wird diese Aufgabe gelöst durch einen medizintechnischen Luftabscheider zum Abscheiden von Gasblasen oder Luftblasen, insbesondere von Mikroblasen, aus durch den Luftabscheider hindurchgeleitetem Blut, aufweisend eine im Wesentlichen hohlzylinderförmige Luftabscheidekammer mit einem in Längsrichtung einerseits der Luftabscheidekammer angeordneten Bluteinlass und einem in Längsrichtung andererseits ausgebildeten Blutauslass, wobei die Luftabscheidekammer derart beschaffen ist, dass eine im Wesentlichen spiralförmig um eine Längsachse der Kammer rotierende Blutströmung vom Bluteinlass zum Blutauslass ausbildbar ist, wobei der Luftabscheider einen angetriebenen Aktor aufweist, der für ein Verstärken der Rotation der spiralförmigen Blutströmung angeordnet und/oder ausgebildet ist. Dabei ist der Bluteinlass auf Seiten eines Kammerdeckels und der Blutauslass auf Seiten eines Kammerbodens angeordnet. Der Aktor weist erfindungsgemäß eine Antriebseinheit für die Luftabscheidekammer auf, um diese entlang einer kreisförmigen oder ellipsenförmigen Bewegungsbahn anzutreiben.

Die Erfindung wird des Weiteren gelöst durch ein Blutschlauchset sowie eine Vorrichtung zur extrakorporalen Blutbehandlung mit einem erfindungsgemäßen Luftabscheider, insbesondere nach einem der abhängigen Ansprüche.

Nach der Erfindung ist die Luftabscheidekammer im Wesentlichen hohlzylinderförmig ausgebildet. Ihre Form kann davon abweichen, sie kann zum Beispiel becherförmig, insbesondere mit einer sich vom Einlass zum Auslass leicht verjüngenden Form ausgebildet sein. Die Luftabscheidekammer ist vorzugsweise um eine Mittellängsachse rotationssymmetrisch und länglich ausgebildet, insbesondere mit einem orthogonal zur Mittellängsachse kreisrundem Querschnitt. Infolge der mechanisch verstärkten Rotation der Blutströmung in der Luftabscheidekammer wird die Verweilzeit von Blut darin erhöht. Man kann sagen, dass die Strömungsbahn des Bluts in der Kammer spiralförmig, wendelförmig oder helixförmig ist, wobei durch den nach der Erfindung zusätzlichen Antrieb mittels des Aktors diese Spirale, Helix oder Wendel stärker gestaucht ist, als bei einem Luftabscheider nach dem Stand der Technik. Im Verlauf der axialen Länge der Luftabscheidekammer führt dadurch strömendes Blut daher eine höhere Anzahl an Umdrehungen um die Mittellängsachse aus als beim Stand der Technik. Es steht daher mehr Zeit zur Verfügung, dass sich in einem bestimmten Blutvolumen vorliegende Blutbestandteile infolge der relativ lange einwirkenden Zentripetalkräfte in einem radial äußeren Bereich des Strudels ansammeln und Luft- oder Gasblasen in einem radial nahe der Mitte des Strudels befindlichen Bereich des rotierenden Bluts ansammeln und abscheiden können. Ein besonderer Vorteil ist, dass derart auch Blasen kleinen Volumens und insbesondere Mikroblasen abgeschieden werden können. Man kann außerdem sagen, dass durch die Verstärkung der Rotationskomponente der Strömung im Strudel neben der höheren Verweilzeit in der Luftabscheidekammer höhere Strömungsgeschwindigkeiten und damit höhere Zentripetalkräfte bewirkt werden, was eine Separation von Blut und Luft- oder Gasblasen fördert.

Der Aktor kann nach einer Ausführungsform der Erfindung unmittelbar auf das in der Kammer befindliche Blut und dessen Strömungsweg wirken. Erfindungsgemäß kann er alternativ oder zusätzlich dazu mittelbar auf das Blut und dessen Strömungsweg wirken, zum Beispiel indem er unmittelbar auf die Luftabscheidekammer wirkt.

Ein weiterer Vorteil der Erfindung ist, dass die die Separation bewirkende Strudelbildung infolge der Wirkung des Aktors weitgehend unabhängig von der jeweils vorliegenden Blutflussrate eingestellt werden kann. Damit kann sowohl bei höheren als auch geringeren Blutflussraten stets eine hohe und sicher erreichbare Abscheidung von Luft/Gas erzielt werden.

Mit der Erfindung können insbesondere die folgenden Vorteile erzielt werden:
- Deutlich verbesserte Abscheidung von Luft- oder Gasblasen, insbesondere von Mikroblasen im Blut,
- zufriedenstellende Funktionen in einem sehr breiten Bereich von Blutflussraten.

Vorteilhafte Ausführungsformen der Erfindung sind in den Unteransprüchen beansprucht und werden nachfolgend näher erläutert.

Bei einer Ausführungsform der Erfindung weist der Aktor ein in der Luftabscheidekammer angeordnetes Rührelement auf. Vorteil dieser Ausführungsform ist, dass der damit ausgebildete Aktor besonders einfach in bekannte Luftabscheider integriert werden kann. Das Rührelement wird derart angetrieben, dass es in dem in der Abscheidekammer vorliegenden Blut eine kreisförmige oder ellipsenförmige Bewegung ausführt, die zwangsläufig auf das Blut übertragen wird. Man kann sagen, dass das Blut in der Abscheidekammer durch simples Rühren in verstärkte Rotation versetzt und derart der vorstehend beschriebene Effekt einer verbesserten Abscheidung von Luft aus Blut bewirkt wird.

Eine Ausführungsform der Erfindung ist dadurch gekennzeichnet, dass das Rührelement magnetisch ist. Ein solches Magnetelement lässt sich einfach durch ein von außen wirkendes Magnetfeld anregen oder antreiben. Eine Felderzeugungseinrichtung zu diesem Zweck ist besonders einfach in eine Vorrichtung zur extrakorporalen Blutbehandlung, insbesondere in eine Dialysemaschine, zu integrieren, was wiederum für eine einfache Integration der Erfindung in bekannte Vorrichtungen Sorge trägt. Das Feld kann mechanisch oder elektrisch erzeugt werden.

Vorzugsweise weist das Rührelement eine abgerundete, kantenlose Geometrie auf. Es kann insbesondere kugelförmig oder stabförmig mit gerundeten Kanten ausgebildet sein. Eine solche Gestalt des Rührelements ist vorteilhaft, da sie für Blutpartikel oder Blutbestandteile schonend ist und somit Hämolyse sicher vermieden werden kann.

Eine weitere Ausführungsform ist dadurch gekennzeichnet, dass das Rührelement biokompatibel ist. Dies kann erreicht werden, indem das gesamte Rührelement aus einem biokompatiblen Material besteht oder einen Kern aus einem bioinkompatiblen Material besitzt, der mit einem biokompatiblen Material beschichtet ist. Das Rührelement kann aus einer biokompatiblen Keramik, insbesondere einer magnetischen Keramik, einem biokompatiblen Kunststoff oder aus einem damit beschichteten Metall bestehen. Außerdem kann das Rührelement hohl sein. Es kann elektrisch leitend und magnetisiert sein, und derart durch Induktion eine Drehkraft erzeugen. Eine Ausführungsform der Erfindung sieht vor, dass das Rührelement eine magnetisierte Kugellagerkugel ist. Dadurch wird der Vorteil erzielt, dass der erfindungsgemäße Luftabscheider sehr günstig in der Herstellung ist, da solche Kugeln günstige Massenprodukte sind.

Es ist von besonderem Vorteil, wenn in der Luftabscheidekammer eine Auflage, ein Anschlag oder ein Positionierelement angeordnet ist, um das Rührelement beabstandet von Bluteinlass und/oder Blutauslass und/oder auf einer bestimmten Höhe zu positionieren oder zu halten. Dadurch kann sichergestellt sein, dass das Rührelement sich stets in einem definierten Bereich der Abscheidekammer befindet und nicht, zum Beispiel bei nicht vorliegendem Antrieb, auf die Unterseite der Kammer absinkt und dort den Blutauslass blockiert, einschränkt oder sperrt. Des Weiteren kann derart sichergestellt werden, dass sich das Rührelement stets in einem Bereich der Kammer befindet, in dem es die gewünschte Rührbewegung ausführen kann und nicht mit Wandungen oder Strukturen der Kammer verklemmt.

Nach der Erfindung weist der Aktor eine Antriebseinheit für die Luftabscheidekammer auf, um diese entlang einer kreisförmigen oder ellipsenförmigen Bewegungsbahn anzutreiben. Bei dieser Ausführungsform ist die gesamte Luftabscheidekammer in Bewegung zu versetzen. Diese ist derart, dass die Rotation des durch die Kammer strömenden Blutstroms verstärkt wird. Man kann auch sagen, dass bei dieser Ausführungsform die Kammer derart geschwenkt wird, dass die Strudelbildung gefördert wird. Beispielsweise kann der Aktor durch eine angetriebene Halterung oder durch eine extern auf die Luftabscheidekammer einwirkende Antriebseinheit gebildet sein.

Der Antrieb der Luftabscheidekammer kann mechanisch erfolgen. Besonders elegant, da sauber und wartungsarm, ist aber, wenn der Aktor magnetisch angetrieben ist. Der erfindungsgemäße Luftabscheider kann zum Antrieb des Aktors insbesondere zumindest zwei Elektromagnete aufweisen, die außerhalb der Luftabscheidekammer angeordnet sind. Der Antrieb kann insbesondere ein um die Luftabscheidekammer rotierendes magnetisches Feld erzeugen.

Die Vorrichtung zur mechanischen Anregung oder zum Antrieb des Aktors kann gleichzeitig als Halter für die Luftabscheidekammer ausgebildet sein. Dies ist besonders raumsparend und wartungsarm. Der Luftabscheider kann des Weiteren eine die Luftabscheidekammer haltende Halteeinrichtung oder Halter aufweisen. Diese kann insbesondere unten, oben oder mittig der Luftabscheidekammer positioniert sein. Der Halter kann insbesondere direkt oder indirekt an einer Vorrichtung zur Anregung befestigt sein oder damit zusammenwirken. Die Anregung kann zum Beispiel mittels eines Elektromotors erfolgen, der den Halter kreisförmig anregt. Der Halter kann zum Beispiel aus Metall bestehen und/oder schwebend gelagert sein. Zur Anregung kann ein solcher Halter durch ein rotierendes Magnetfeld bewegt werden.

Es besteht die Möglichkeit, dass die Drehzahl des Aktors und/oder die Energie zum Antrieb des Aktors sensorisch erfasst wird, insbesondere in Abhängigkeit von der Drehzahl des Aktors, und daraus die Viskosität des durch den Luftabscheider geleiteten Bluts bestimmt wird. Zur Erfassung der Drehzahl des Aktors kann bspw. ein Magnetfeldsensor genutzt werden und vorhanden sein. Durch eine Messung der zur Rotation mit einer bestimmten Drehzahl erforderlichen Kraft, zum Beispiel zu erfassen über die dabei benötigte elektrische Energie, kann auf die Viskosität des Blutes geschlossen werden. Wird diese über die Laufzeit der Behandlung erfasst, so lassen sich daraus Rückschlüsse auf das relative Blutvolumen ziehen. Dieser Parameter kann zur Regelung z.B. der Ultrafiltrationsmenge herangezogen werden. Zusätzlich oder alternativ kann die Drehzahl des Aktors blutflussabhängig geregelt werden. Als Rotationsfrequenz des Aktors sind in vorteilhafter Weise wenige Hz, vorzugsweise 2-10 Hz. bei einer Auslenkung von 3-10mm ausreichend, um die gewünschte Separation von Luftblasen zu erzielen. Dies ist insbesondere vorteilhaft, da schonend für das Blut, wodurch Hämolyse sicher vermieden werden kann. Außerdem ist es möglich, dass alternativ größere Frequenzen von 10-50 Hz bei kleiner Auslenkung von 1-2mm zur Anwendung kommen. Beide Parameter sind auf die jeweils vorliegenden Kammergeometrien und/oder Kammerabmessungen angepasst und optimiert.

Außerdem kann zur Bestimmung der Viskosität des durch den Luftabscheider hindurchgeleiteten Bluts der Antrieb des Aktors unterbrochen oder eingestellt werden, insbesondere nach Erreichen einer bestimmungsgemäßen Messdrehzahl, das Nachlaufverhalten des Aktor erfasst werden und die Viskosität anhand des Nachlaufverhaltens bestimmt werden. Insbesondere kann die Viskosität gemessen werden, indem das elektrische Feld zum Antrieb des Aktors unterbrochen wird, nachdem dieser auf eine bestimmte Drehzahl gebracht wurde. Aus der dann erzeugten Induktion während des Abbremsens kann die Dauer des Abbremsvorgangs gemessen und damit auf die Viskosität geschlossen werden.

Es ist von Vorteil, dass anhand einer Viskositätsmessung Rückschlüsse gezogen werden können, ob das Rührelement noch vollständig "unter Blut" ist, so dass auf diese Weise eine Luftpegelerkennung realisiert wird. Dadurch kann verhindert werden, dass ein Sensor, der nach der Kammer angebracht ist (Luftblasendetektor), erst anspricht, wenn die Kammer leer ist und Luft bereits bis hinter den Sensor gelangt ist. Die Beseitigung der Störung ist dann wesentlich aufwändiger, als wenn rechtzeitig erkannt wird, wenn der Pegel absinkt.

Man kann auch sagen, dass nach der Erfindung vorgeschlagen wird, über einen angetriebenen Aktor, wie einen Magnetrührer, in einem Luftabscheider eines Blutschlauchsets für eine Dialysemaschine eine bessere Luftabscheidung zu erreichen. Dazu kann nach der Erfindung in die Luftabscheidekammer ein magnetisches Element eingebracht werden, um die Flüssigkeit in Rotation zu versetzen. Aufgrund der dabei wirkenden Zentrifugalkräfte kann sich Blut außen (am radial äußeren Randbereich der Strömung) ansammeln und Luft in der Mitte bleiben. Es entsteht ein Strudel. Das magnetische Element kann insbesondere mittels eines rotierenden Magnetfelds von außen in Rotation versetzt werden, das von zumindest zwei Elektromagneten erzeugt werden kann. Diese können in einem Halter für die Luftabscheidekammer eingebaut sein. Alternativ kann das Feld mechanisch durch rotierende Magnete erzeugt werden. Nach einer Ausführungsform kann eine Erzeugung des Strudels durch eine mechanische Anregung des Luftabscheiders erfolgen. Dabei kann zum Beispiel eine Seite des Luftabscheiders auf einer kreisförmigen oder elliptischen Bahn bewegt oder zu einer derartigen Bewegung angeregt werden, während seine andere Seite an einem beweglichem Fixpunkt gelagert ist, so dass ein Strudel im Luftabscheider erzeugt wirkt. Der Luftabscheider selbst dreht sich nicht um seine Achse.

### Kurzbeschreibung der Figuren

Die Erfindung wird im Folgenden anhand beispielhafter, nicht einschränkender und in den angehängten Figuren gezeigter Ausführungsformen näher erläutert. Dabei zeigt:
Figur 1 eine schematische Schnittansicht entlang einer Mittellängsachse eines medizinischen Luftabscheiders nach dem Stand der Technik,
Figur 2 eine schematische Schnittansicht entlang der Mittellängsachse eines medizinischen Luftabscheiders nach einer Ausführungsform der Erfindung, ohne die Antriebseinheit zur Rotation der Luftabscheidekammer selbst zu zeigen,
Figur 3 eine schematische Schnittansicht entlang der Mittellängsachse des medizinischen Luftabscheiders der Figur 1 mit einer Magneteinheit zur Anregung des Aktors, ohne die Antriebseinheit zur Rotation der Luftabscheidekammer selbst zu zeigen,
Figur 4 eine Schnittansicht orthogonal zur Mittellängsachse entlang der in Figur 3 eingezeichneten Schnittlinie IV-IV,
Figur 5 eine schematische Schnittansicht entlang der Mittellängsachse eines medizinischen Luftabscheiders nach einer weiteren Ausführungsform der Erfindung, ohne die Antriebseinheit zur Rotation der Luftabscheidekammer selbst zu zeigen,
Figur 6 eine schematische Schnittansicht entlang der Mittellängsachse eines medizinischen Luftabscheiders nach einer weiteren Ausführungsform der Erfindung, ohne die Antriebseinheit zur Rotation der Luftabscheidekammer selbst zu zeigen,
Figur 7 eine schematische Schnittansicht entlang der Mittellängsachse eines medizinischen Luftabscheiders nach einer weiteren Ausführungsform der Erfindung, ohne die Antriebseinheit zur Rotation der Luftabscheidekammer selbst zu zeigen,
Figur 8 eine schematische Schnittansicht entlang der Mittellängsachse eines medizinischen Luftabscheiders nach einer weiteren Ausführungsform der Erfindung,
Figur 9 eine schematische Schnittansicht entlang der Mittellängsachse eines medizinischen Luftabscheiders nach einer weiteren Ausführungsform der Erfindung,
Figur 10 eine schematische Schnittansicht entlang der Mittellängsachse eines medizinischen Luftabscheiders nach einer weiteren Ausführungsform der Erfindung,
Figur 11 eine schematische Schnittansicht entlang der Mittellängsachse eines medizinischen Luftabscheiders nach einer weiteren Ausführungsform der Erfindung,
Figur 12 eine Schnittansicht orthogonal zur Mittellängsachse entlang der in Figur 3 eingezeichneten Schnittlinie IV-IV und
Figur 13 eine vereinfachte Darstellung der Figur 12.

In der Figurenbeschreibung werden gleiche oder entsprechende Elemente mit identischen Bezugszeichen versehen.

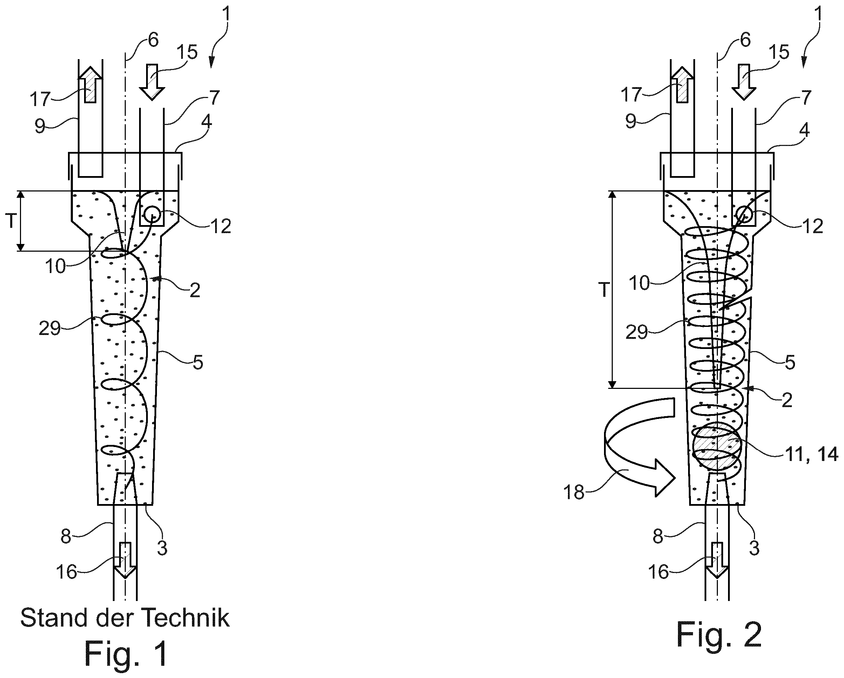

Figur 1 zeigt einen medizintechnischen Luftabscheider 1 zum Abscheiden von Gasblasen nach dem Stand der Technik. Dieser weist eine Luftabscheidekammer 2 mit einem Kammerboden 3, einem Kammerdeckel 4 und einer zylinder- oder becherförmigen Kammerwand 5 auf. Eine Mittellängsachse 6 der Luftabscheidekammer 2 ist in Figur 1 gekennzeichnet. Auf Seiten des Kammerdeckels 4 ist ein Bluteinlass 7 für einen in die Kammer 2 hineinströmenden Blutzustrom 15 angeordnet. Auf Seiten des Kammerbodens 3 ist ein Blutauslass 8 für einen aus der Kammer 2 herausströmenden Blutabstrom 16 angeordnet. Über den Bluteinlass 7 wird Blut mit einer seitlichen oder tangentialen Einströmrichtung in die Luftabscheidekammer 2 eingeleitet. Dies ergibt sich aus der Darstellung einer Austrittsöffnung 12 für Blut aus dem Bluteinlass 7. Infolge der seitlichen Einströmung wird in der Kammer 2 ein Strudel 10 erzeugt. Das Blut strömt in einer spiralförmigen oder wendelförmigen Bahn 29 vom Bluteinlass 7 zum Blutauslass 8. Infolge der bei einem derartigen Strömungsbild 29 wirkenden Zentrifugalkräfte bzw. Zentripetalkräfte wird Blut nach radial außen gedrückt, während Luft und damit auch Luft- oder Gasblasen in der Mitte (nahe der Mittellängsachse 6) der rotierenden Strömung 10 und der Kammer 2 bleiben. Sich randseitig des Strudels 10 ansammelnde Luft wird als Abluftstrom 17 über eine Entlüftungsöffnung 9 aus der Kammer 2 geleitet. Bei dem Luftabscheider nach dem Stand der Technik werden die Strömung 29 und der Strudel 10 in der Kammer 2 allein durch die tangentiale Einleitung des Bluts erzeugt. Der sich ausbildende Strudel 10 ist daher, wie sich aus einem Vergleich der Figuren 1 und 2 ergibt, relativ klein und die spiralförmige Strömungsbahn 29 relativ gestreckt. Man kann sagen, dass sich allein durch das vorstehend beschriebene, alleinige gerichtete (seitlich oder tangentiale) Einströmen ein nur leichter Strudel 10 von geringer Tiefe T ausbildet.

Figur 2 zeigt eine erste Ausführungsform eines medizintechnischen Luftabscheiders 1 zum Abscheiden von Gasblasen, insbesondere von Mikrogasblasen, aus durch den Luftabscheider 1 hindurchgeleitetem Blut. Dabei wird die Antriebseinheit zur Rotation der Luftabscheidekammer selbst nicht gezeigt. Der Luftabscheider 1 weist eine im Wesentlichen hohlzylinderförmige oder becherförmige Luftabscheidekammer 2 mit einem Kammerboden 3, einem Kammerdeckel 4 und einer zylinder- oder becherförmigen Kammerwand 5 auf. Die Mittellängsachse 6 der Luftabscheidekammer 2 ist in Figur 2 gekennzeichnet. In Längsrichtung 6 einerseits der Luftabscheidekammer 2, nämlich auf Seiten des Kammerdeckels 4, ist der Bluteinlass 7 für den Blutzustrom 15 angeordnet. Im vorliegenden Ausführungsbeispiel ist er in dem Kammerdeckel 4 ausgebildet. In Längsrichtung 6 andererseits, nämlich auf Seiten des Kammerbodens 3, ist der Blutauslass 8 für den Blutabstrom 16 angeordnet. Im vorliegenden Ausführungsbeispiel ist er mittig im Kammerboden 3 ausgebildet. Im Kammerdeckel 4 ist des Weiteren die Entlüftungsöffnung 9 für aus dem Blut abgeschiedene Luft als Abluftstrom 17 vorgesehen.

Die Luftabscheidekammer 1 ist derart beschaffen, dass wiederum eine im Wesentlichen spiralförmig um die Mittellängsachse 6 der Kammer 2 rotierende Blutströmung 29 vom Bluteinlass 7 zum Blutauslass 8 und damit ein Strudel 10 ausgebildet wird. Aus einem Vergleich der Figuren 1 und 2 ergibt sich deutlich, dass der Strudel 10 bei dem Luftabscheider 1 nach der Erfindung deutlich stärker, das heißt mit einer größeren Tiefe T, ausgeprägt ist als bei dem Luftabscheider 1 nach dem Stand der Technik. Die Strömungsbahn 29 ist deutlich stärker gestaucht als beim Stand der Technik. Dies ist dadurch begründet, dass der erfindungsgemäße Luftabscheider 1 einen angetriebenen Aktor 11 aufweist. Dieser dient der in der Figur gezeigten Verstärkung der Rotation der spiralförmigen Blutströmung 29. Infolge der durch den angetriebenen Aktor 11 bewirkten vermehrten Rotation der Strömung 29 in der Kammer 2 wirken auf das Blut höhere Zentripetalkräfte, so dass vermehrt Blut nach radial außen gedrängt wird und der Strudel 10 ausgeprägter und tiefer als beim Stand der Technik ausgebildet wird. Man kann sagen, dass sich durch den zusätzlichen angetriebenen Aktor 11 nach der Erfindung ein tiefer Strudel 10 mit entsprechend starker Luftabscheidung ausbildet. Bei der Ausführungsform der Figur 2 ist am Blutauslass 8 ein Filterelement 13, hier in Form eines Koagelfilters 13 angeordnet.

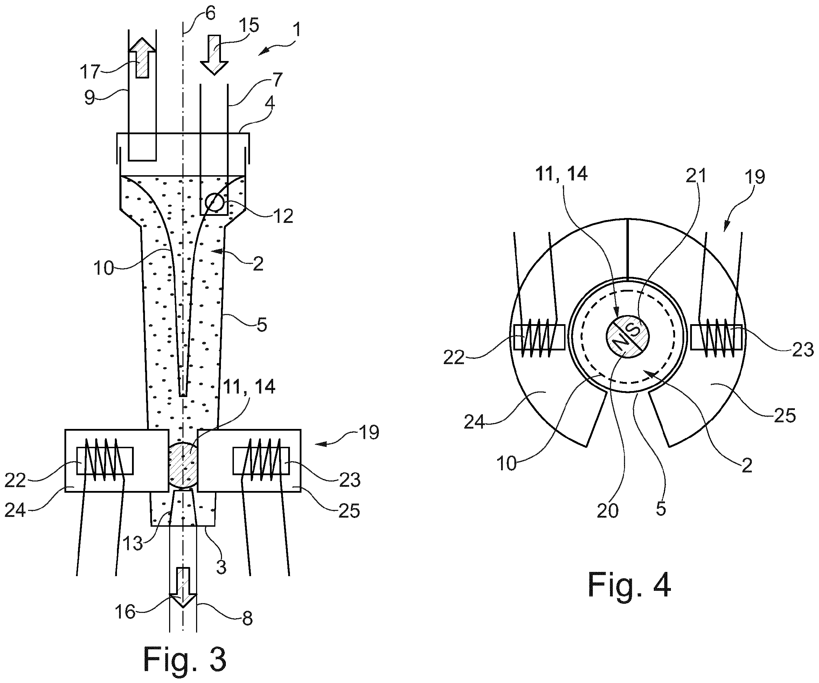

Nach einer Ausführungsform der Erfindung kann der Aktor 11 durch ein in der Kammer 2 angeordnetes Rührelement 14 in Form zum Beispiel einer Kugel 14 oder eines Stabelements 14 gebildet sein. Dieses führt in der Luftabscheidekammer 2 infolge einer mittels eines Pfeils 18 angedeuteten Anregung durch einen in Figur 2 nicht gezeigten Antrieb eine Bewegung auf einer Kreisbahn aus. Infolge dieser Bewegung wird das in der Kammer 2 befindliche und diese durchströmende Blut zusätzlich rotatorisch angetrieben. Die Figuren 3 und 4 verdeutlichen den Antrieb 19 für das Rührelement 14. Dieses ist als Magnetkugel 14 mit einer Nordhälfte 20 und einer Südhälfte 21 ausgebildet. Der Antrieb 19 weist zwei einander mit Bezug auf die Mittellängsachse 6 diametral gegenüberliegende Magnetspulen 22, 23 auf, die mit Kernen 24, 25 zusammenwirken. Der Antrieb 19 bildet ein um die Mittellängsachse 6 rotierendes Magnetfeld aus, dessen Wirkung auf die Magnetkugel 14 deren vorstehend beschriebene kreis- oder ellipsenförmige Bewegung in der Luftabscheidekammer 2 hervorruft.

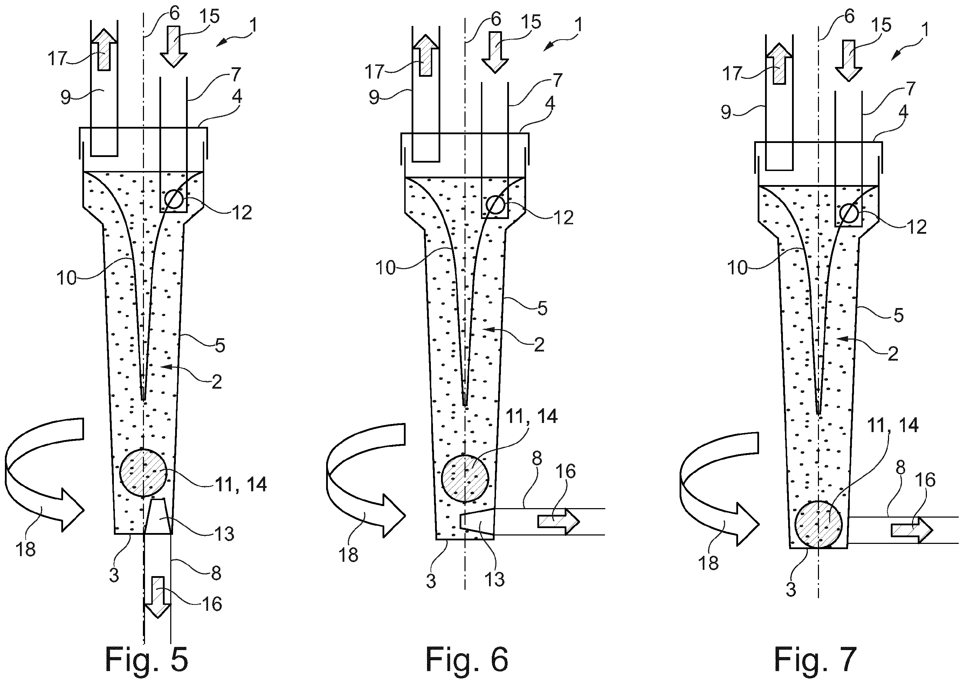

Die Ausführungsform der Figur 5 entspricht im Wesentlichen der der Figuren 2 bis 4, so dass auf die diesbezügliche Beschreibung verwiesen wird. Anders als bei der Ausführungsform der Figuren 2 bis 4 ist aber der Koagelfilter 13 nicht mittig des Kammerbodens 3, sondern in dessen Randbereich außermittig zur Kammerwand 5 versetzt angeordnet. Dies bewirkt den Vorteil, dass Blut mit einer höheren Strömungsgeschwindigkeit in umfänglicher Richtung auf den Filter 13 trifft und ein besseres Ausströmverhalten erzeugt wird. Ein ähnlicher Effekt tritt in der Ausführungsform der Figur 6 auf, bei der der Koagelfilter 13 benachbart zum Kammerboden 3 in der Kammerwand 5 angeordnet ist. Figur 7 zeigt eine der Figur 6 entsprechende Ausführungsform ohne Filter.

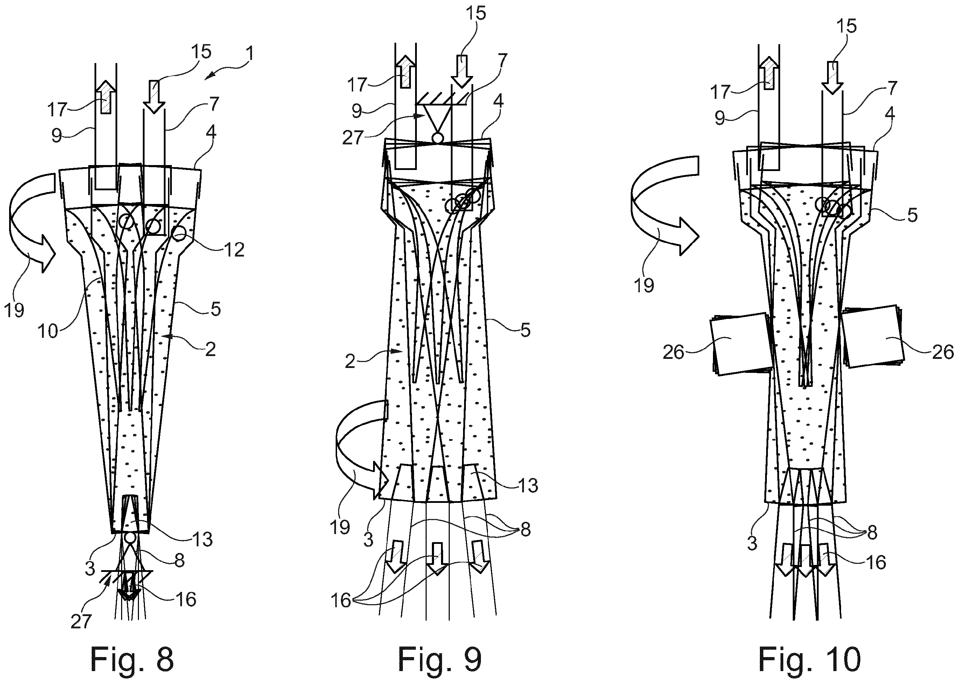

Die Figuren 8 bis 13 zeigen Ausführungsformen der Erfindung, bei denen der Aktor 11 als Antriebseinheit 26 für die Luftabscheidekammer 2 ausgebildet ist. Es ist zu beachten, dass diese Figuren eine Überlagerung von mehreren bei einer Aktuierung des Luftabscheiders zeitlich aufeinanderfolgenden Positionen darstellen. Es sei darauf hingewiesen, dass die Funktionsprinzipien der vorliegend beschriebenen Ausführungsformen kombiniert werden können, also ein Aktor 11 der Ausführungsformen der Figuren 8 bis 13 mit einem Rührelement der Figuren 2 bis 7 kombiniert verwendet werden kann. Die Antriebseinheit 26 ist aus Gründen einer einfachen Darstellung in den Figuren 8, 9 und 11 nicht gezeigt. Sie kann insbesondere gemäß der Ausführungsform der Figur 10 als Halter 26 ausgebildet sein, der die Kammerwand 5 haltend kontaktiert. Die Antriebseinheit 26 / der Halter 26 wirkt mit der Luftabscheidekammer 2 zusammen und treibt diese derart an, dass sie sich entlang einer kreisförmigen oder ellipsenförmigen Bewegungsbahn bewegt.

Figur 8 zeigt den erfindungsgemäßen Luftabscheider 1, bei dem die Luftabscheidekammer 2 am Kammerboden 3 von einer schematisch angedeuteten Lagereinheit 27 ortsfest aber beweglich gelagert ist. Die Luftabscheidekammer 2 führt infolge der Lagerung mit der Lagereinheit 27 und des Antriebs 26 / des Aktors 11 eine Art Taumel- oder Kreiselbewegung um die Mittellängsachse 6 aus, wobei die Lagereinheit 27 einen Fixpunkt der Kreiselbewegung definiert. Figur 9 zeigt eine ähnliche Ausführungsform, bei der die Lagereinheit mittig am Kammerdeckel 4 befindlich ist und die Taumel- oder Kreiselbewegung demnach quasi umdreht. Figur 10 zeigt eine Ausführungsform ohne Lagereinheit, bei der der Halter 26 gleichzeitig antreibt und in etwa mittig der Längsachse 6 einen Fixpunkt definiert.

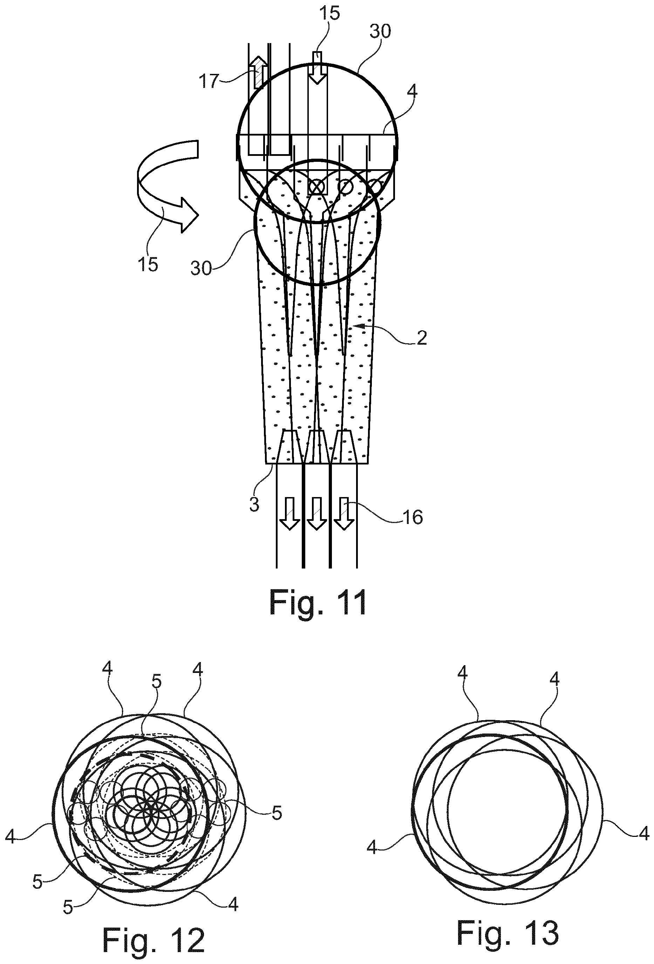

Die Figuren 11 bis 13 zeigen eine weitere Ausführungsform, bei der die gesamte Luftabscheidekammer 2 eine Bewegung auf einer Kreisbahn ausführt. Die Figuren 11, 12 und 13 verdeutlichen dabei die Positionen der Kammer 2 zu vier unterschiedlichen Zeitpunkten während der Kreisbewegung.

### Bezugszeichen

- 1: Luftabscheider
- 2: Luftabscheidekammer
- 3: Kammerboden
- 4: Kammerdeckel
- 5: Kammerwand
- 6: Mittellängsachse, Längsrichtung
- 7: Bluteinlass
- 8: Blutauslass
- 9: Entlüftung
- 10: Strudel
- 11: Aktor
- 12: Austrittsöffnung
- 13: Filter, Koagelfilter
- 14: Rührelement, Kugel, Stabelement
- 15: Blutzustrom
- 16: Blutabstrom
- 17: Abluftstrom
- 18: Pfeil (verdeutlicht Anregung)
- 19: Antrieb
- 20: Nordhälfte
- 21: Südhälfte
- 22: Magnetspule
- 23: Magnetspule
- 24: Kern
- 25: Kern
- 26: Antriebseinheit, Halter
- 27: Lagereinheit
- 29: Strömungsbahn
- T: Tiefe des Strudels 10

## Patentansprüche

1. Medizintechnischer Luftabscheider (1) zum Abscheiden von Gasblasen, insbesondere von Mikrogasblasen, aus durch den Luftabscheider (1) hindurchgeleitetem Blut, aufweisend eine im Wesentlichen hohlzylinderförmige Luftabscheidekammer (2) mit einem in Längsrichtung (6) einerseits der Luftabscheidekammer (2) angeordneten Bluteinlass (7) und einem in Längsrichtung (6) andererseits ausgebildeten Blutauslass (8), wobei die Luftabscheidekammer (2) derart beschaffen ist, dass eine im Wesentlichen spiralförmig um eine Längsachse (29) der Kammer (2) rotierende Blutströmung (29) vom Bluteinlass (7) zum Blutauslass (8) ausbildbar ist, wobei
der Luftabscheider (1) einen angetriebenen Aktor (11) aufweist zum Verstärken der Rotation der spiralförmigen Blutströmung (29); und
der Bluteinlass (7) auf Seiten eines Kammerdeckels (4) und der Blutauslass (8) auf Seiten eines Kammerbodens (3) angeordnet sind, **dadurch gekennzeichnet, dass** der Aktor (11) eine Antriebseinheit (26) für die Luftabscheidekammer (2) aufweist, um diese entlang einer kreisförmigen oder ellipsenförmigen Bewegungsbahn (30) anzutreiben.

2. Luftabscheider (1) nach Anspruch 1, **dadurch gekennzeichnet, dass** der Aktor (11) ein in der Luftabscheidekammer (2) angeordnetes Rührelement (14) aufweist.

3. Luftabscheider (1) nach Anspruch 2, **dadurch gekennzeichnet, dass** das Rührelement (14) magnetisch ist und/oder eine abgerundete, kantenlose Geometrie aufweist, insbesondere kugelförmig oder stabförmig mit gerundeten Kanten ausgebildet ist.

4. Luftabscheider (1) nach Anspruch 2 oder 3, **dadurch gekennzeichnet, dass** das Rührelement (14) biokompatibel ist, insbesondere aus einer biokompatiblen Keramik, einem biokompatiblen Kunststoff oder aus einem damit beschichteten Metall besteht.

5. Luftabscheider (1) nach einem der vorstehenden Ansprüche 2 bis 4, **dadurch gekennzeichnet, dass** in der Luftabscheidekammer (2) eine Auflage (13) angeordnet ist, um das Rührelement (14) beabstandet von Bluteinlass (7) und/oder Blutauslass (8) zu positionieren.

6. Luftabscheider (1) nach einem der vorstehenden Ansprüche, **dadurch gekennzeichnet, dass** der Aktor (11) magnetisch angetrieben ist.

7. Luftabscheider (1) nach einem der vorstehenden Ansprüche, des Weiteren aufweisend zumindest zwei Elektromagnete (22, 23, 24, 25), die außerhalb der Luftabscheidekammer (2) angeordnet sind, zum Antrieb des Aktors (11).

8. Luftabscheider (1) nach einem der vorstehenden Ansprüche, des Weiteren aufweisend eine die Luftabscheidekammer (2) haltende Halteeinrichtung (26).

9. Blutschlauchset mit einem Luftabscheider (1) nach einem der vorstehenden Ansprüche.

10. Vorrichtung zur extrakorporalen Blutbehandlung mit einem Luftabscheider (1) nach einem der vorstehenden Ansprüche.

11. Vorrichtung zur extrakorporalen Blutbehandlung nach Anspruch 10, aufweisend Mittel zum Erfassen der Drehzahl des Aktors (11) und/oder Mittel zur Erfassung der Energie zum Antrieb des Aktors (11), insbesondere in Abhängigkeit von der Drehzahl des Aktors (11).

## Claims

1. A medical air separator (1) for separating gas bubbles, especially micro gas bubbles, from blood passed through the air separator (1), comprising a substantially hollow-cylindrical air separating chamber (2) having a blood inlet (7) disposed on one side of the air separating chamber (2) viewed in the longitudinal direction (6), and a blood outlet (8) formed on the other side viewed in the longitudinal direction (6), wherein the air separating chamber (2) is configured so that a blood flow (29) rotating about a longitudinal axis (29) of the chamber (2) substantially in spiral shape can be formed from the blood inlet (7) to the blood outlet (8),
wherein
the air separator (1) comprises a driven actuator (11) for increasing rotation of the spiral-shaped blood flow (29); and
the blood inlet (7) is arranged on the side of a chamber cover (4) and the blood outlet (8) is arranged on the side of a chamber bottom (3), **characterized in that**
the actuator (11) comprises a drive unit (26) for the air separating chamber (2) so as to drive the latter along a circular or elliptic path of motion (30).

2. The air separator (1) according to claim 1, **characterized in that** the actuator (11) comprises a stirring element (14) disposed in the air separating chamber (2).

3. The air separator (1) according to claim 2, **characterized in that** the stirring element (14) is magnetic and/or has a rounded edgeless geometry, is formed especially ball-shaped or rod-shaped with rounded edges.

4. The air separator (1) according to claim 2 or 3, **characterized in that** the stirring element (14) is biocompatible, and is especially made from biocompatible ceramics, biocompatible plastic or metal coated therewith.

5. The air separator (1) according to one of the preceding claims 2 to 4, **characterized in that** in the air separating chamber (2) a support (13) is arranged to position the stirring element (14) spaced apart from the blood inlet (7) and/or the blood outlet (8).

6. The air separator (1) according to one of the preceding claims, **characterized in that** the actuator (11) is magnetically driven.

7. The air separator (1) according to one of the preceding claims, further comprising at least two solenoids (22, 23, 24, 25) disposed outside the air separating chamber (2) for driving the actuator (11).

8. The air separator (1) according to one of the preceding claims, further comprising a holding device (26) holding the air separating chamber (2).

9. A blood tubing set comprising an air separator (1) according to one of the preceding claims.

10. An apparatus for extracorporeal blood treatment comprising an air separator (1) according to one of the preceding claims.

11. The apparatus for extracorporeal blood treatment according to claim 10, comprising means for detecting the speed of the actuator (11) and/or means for detecting the energy for driving the actuator (11), especially in response to the speed of the actuator (11).

## Revendications

1. Séparateur d'air médical (1) pour séparer des bulles de gaz, en particulier des microbulles de gaz, du sang passant à travers le séparateur d'air (1), présentant une chambre de séparation d'air (2) sensiblement en forme de cylindre creux avec une entrée de sang (7) agencée dans la direction longitudinale (6) d'une part de la chambre de séparation d'air (2) et une sortie de sang (8) réalisée dans la direction longitudinale (6) d'autre part, dans lequel la chambre de séparation d'air (2) est conçue de sorte qu'un écoulement de sang (29) tournant sensiblement en forme de spirale autour d'un axe longitudinal (29) de la chambre (2) peut être formé de l'entrée de sang (7) à la sortie de sang (8), dans lequel
le séparateur d'air (1) présente un actionneur entraîné (11) pour amplifier la rotation de l'écoulement de sang (29) en forme de spirale ; et
l'entrée de sang (7) est agencée sur les côtés d'un couvercle de chambre (4) et la sortie de sang (8) est agencée sur les côtés d'un fond de chambre (3), **caractérisé en ce que**
l'actionneur (11) présente une unité d'entraînement (26) pour la chambre de séparation d'air (2) afin d'entraîner celle-ci le long d'une trajectoire de déplacement (30) de forme circulaire ou elliptique.

2. Séparateur d'air (1) selon la revendication 1, **caractérisé en ce que** l'actionneur (11) présente un élément d'agitation (14) agencé dans la chambre de séparation d'air (2).

3. Séparateur d'air (1) selon la revendication 2, **caractérisé en ce que** l'élément d'agitation (14) est magnétique et/ou présente une géométrie arrondie, sans arêtes, en particulier est réalisé en forme de sphère ou de barre avec des arêtes arrondies.

4. Séparateur d'air (1) selon la revendication 2 ou 3, **caractérisé en ce que** l'élément d'agitation (14) est biocompatible, en particulier est constitué d'une céramique biocompatible, d'une matière plastique biocompatible ou d'un métal revêtu de celle-ci.

5. Séparateur d'air (1) selon l'une quelconque des revendications 2 à 4 précédentes, **caractérisé en ce qu'**un support (13) est agencé dans la chambre de séparation d'air (2) afin de positionner l'élément d'agitation (14) à distance de l'entrée de sang (7) et/ou de la sortie de sang (8).

6. Séparateur d'air (1) selon l'une quelconque des revendications précédentes, **caractérisé en ce que** l'actionneur (11) est entraîné magnétiquement.

7. Séparateur d'air (1) selon l'une quelconque des revendications précédentes, présentant en outre au moins deux électroaimants (22, 23, 24, 25) agencés à l'extérieur de la chambre de séparation d'air (2) pour entraîner l'actionneur (11).

8. Séparateur d'air (1) selon l'une quelconque des revendications précédentes, présentant en outre un appareil de maintien (26) maintenant la chambre de séparation d'air (2).

9. Ensemble de tubes à sang avec un séparateur d'air (1) selon l'une quelconque des revendications précédentes.

10. Dispositif de traitement extracorporel du sang avec un séparateur d'air (1) selon l'une quelconque des revendications précédentes.

11. Dispositif de traitement extracorporel du sang selon la revendication 10, présentant des moyens de détection de la vitesse de rotation de l'actionneur (11) et/ou des moyens de détection de l'énergie pour l'entraînement de l'actionneur (11), en particulier en fonction de la vitesse de rotation de l'actionneur (11).
